Europäisches Patentamt

European Patent Office

Office européen des brevets

(19)

(11) Publication number: **0 096 674**
**B1**

(12) **EUROPEAN PATENT SPECIFICATION**

(45) Date of publication of patent specification: **05.03.86**

(51) Int. Cl.⁴: **C 12 Q 1/06**

(21) Application number: **83850154.2**

(22) Date of filing: **03.06.83**

(54) **Method for pre-treatment of samples containing in addition to microorganisms other biological material.**

(30) Priority: **09.06.82 SE 8203564**

(43) Date of publication of application:
**21.12.83 Bulletin 83/51**

(45) Publication of the grant of the patent:
**05.03.86 Bulletin 86/10**

(84) Designated Contracting States:
**BE DE FR GB IT LU NL**

(56) References cited:
**EP-A-0 025 351**
**GB-A-2 059 990**
**US-A-4 212 948**
**CHEMICAL ABSTRACTS, vol. 89, no. 15, 9th
October 1978, page 275, no. 125662m,
Columbus, Ohio, USA H. PERTOFT et al.:
"Density gradients prepared from colloidal
silica particles coated by polyvinylpyrrolidone
(Percoll)"**
**CHEMICAL ABSTRACTS, vol. 82, no. 25, 23rd
June 1975, page 223, no. 166972m, Columbus,
Ohio, USA A. THORE et al.: "Detection of
bacteriuria by luciferase assay of adenosine
triphosphate"**

(73) Proprietor: **LKB-Produkter AB
Box 305
S-161 26 Bromma (SE)**
(73) Proprietor: **Nilsson, Lennart
Björknäs Nykil
S-590 50 Vikingstad (SE)**
(73) Proprietor: **Molin, Örjan
c/o Rehnström Nybrogatan 68
S-114 41 Stockholm (SE)**

(72) Inventor: **Nilsson, Lennart
Björknäs Nykil
S-590 50 Vikingstad (SE)**
Inventor: **Molin, Örjan
c/o Rehnström Nybrogatan 68
S-114 41 Stockholm (SE)**
Inventor: **Lundin, Arne
Gaveliusgatan 6
S-116 41 Stockholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention refers to a method for pre-treatment of samples containing microorganisms and other biological material as a preliminary step in a rapid analysis of the microorganisms.

Quantification of microorganisms in samples containing also other biological material is a common analytical problem. It could be e.g. the presence of bacteria in clinical samples (blood, liquor, wound discharge, tissue and urine) or food samples (milk, tinned goods and cured meats).

Traditional microbiological analysis is based on the growth of micoorganisms and is therefore slow. The determination of bacteria in a sample could be mentioned as an example. In this determination the sample is brought in contact with a growth medium after which an incubation follows during which the bacteria are multiplying to numbers visible to the eye. The method is thus based on the fact what the amount of bacteria increases while the amount of other biological material is constant. Thus, the sensitivity as well as the specificity become very good. The drawback of the method is the long incubation time required for growth (incubation over night on in some cases for several days).

One has for a long time tried to work out less time-consuming methods for the analysis of microorganisms based on chemical or physical methods for analysis. One alternative is to measure the growth of microorganisms in a more sensitive way than that which is used in traditional microbiological analysis. As a certain time is often required before the growth starts (the so-called lag-phase) the saving of time is, however, limited in this method. Another alternative is to measure by means of sensitive analytical techniques constituents of microorganisms either naturally present in the microorganisms or in an artificial way associated with the microorganisms (e.g. radioactive markers absorbed by the microorganisms or bound to the microorganisms by means of e.g. specific antibodies). The sensitivity in a rapid analysis of microorganisms based on the measurement of constituents of microorganisms is often adequate. In a luminescence analysis the amount of adenosinetriphosphate (ATP) could for example be measured from 1000 cells of bacteria or in some cases from fewer cells. In the presence of other biological material the specificity problems are often important as it is difficult to find sensitive analysis methods for the few microorganism constituents which are completely specific for the microorganisms. This is especially valid as microorganisms are small compared to e.g. blood cells. Thus, one single red blood cell contains 30—60 times as much ATP as a bacterial cell (Thore, A., Ånséhn, S., Lundin, A. and Bergman, S. (1975) J. Clin. Microbiol, 1, 1—8)).

To solve the above specificity problem a number of principles for separation of microorganisms from other biological material has been tested. Thus, it would be possible to separate microorganisms from other cells using fractionated centrifugation based on the differences of the sedimentation velocity of the cells or filtration based on the differences in the size of the cells. However, in practice it appears that these principles are not often valid. The separation is often poor due to e.g. the fact that the microorganisms are associated to other biological material. In other cases the amounts of cell constituents, of the microorganisms are influenced during the separation so that the analysis becomes uncertain. This is valid for e.g. measurement of microbiological ATP after the collection of the microorganisms by means of fractionated centrifugation or filtration (Lundin, A. and Thore, A. (1975) Appl. Microbiol. 30, 713—721). In certain cases the separation cannot be performed for purely technical reasons (it is e.g. very difficult to filter certain samples when the pores of the membrane filters are clogged).

In one case one has succeeded in solving the above specificity problem by means of a specific chemical pre-treatment of the sample. Bacteria in urine can thus be detected through luminescence analysis of bacterial ATP after pre-treatment of the sample with Triton X-100, a detergent which lyses somatic cells but not bacterial cells, and apyrase, an enzyme which decomposes free ATP but which does not affect ATP in bacterial cells (Thore, A., Ånséhn, S., Lundin, A. and Bergman, S. (1975) J. Clin. Microbiol, 1, 1—8). However, this method is only valid if the amount of other biological material is relatively limited. Thus, it is not valid for analysis of bacteria in e.g. blood, wound discharges, milk and cured meats.

Thus, the situation could be summarized as follows: Except for the analysis of bacteria in urine there are no general methods applicable to rapid analysis of microorganisms in samples containing also other biological material.

The object of the present invention is to provide a method to make it possible to separate microorganisms from other biological material so that the analysis of naturally present or artificially associated constituents of microorganisms could be used for rapid analysis of microorganisms in samples containing also high amounts of other biological material, e.g. blood. According to the invention the separation of microorganisms from other biological material is based on the equilibrium centrifugation in a density gradient (below called gradient centrifugation). Gradient centrifugation, i.e. separation of sample components based on density differences, is a well known technique which has existed for many years (for a summary of the method and applications, see e.g. Percoll®, Methodology and applications. Density Marker Beads. For calibration of gradients of Percoll®. Pharmacia Fine Chemicals AB, Box 175, S-751 04 Uppsala 1, 1980).

Separation of microorganisms from other biological material using gradient centrifugation would be a possible preliminary preparation of the sample in connection with rapid analysis of microorganisms in biological samples. However, in practice it is found that the separation often is not good enough to obtain an adequate specificity and sensitivity. For example, bacteria and certain blood cells have about the same density and therefore this methodology cannot be used in quantification of bacteria in blood.

The invention refers to a method which makes possible adequate specificity and sensitivity in rapid analysis of microorganisms in samples containing also high amounts of other biological material. According to the invention this is achieved by means of a combination of gradient centrifugation and treatment of the sample using agents which specifically lyse non - microbiological cells.

The combination of gradient centrifugation and specifically lysing agents has the following advantages:

1. The separation of microorganisms from other biological material becomes better after lysis of non - microbiological cells, as the cell fragments obtained from the non - microbiological cells have densities which differ more from the density of the microorganisms.

2. By the lysis the non - microbiological pool of the substances used in the quantification of the microorganisms is set free. The non - microbiological pool could thus be easier decomposed or in some other way be removed before the quantification of the microbiological pool.

3. Intracellular microorganisms will be susceptible to rapid analysis.

4. Gradient centrifugation makes possible the concentration of the microorganisms as compared to the original sample, which improves the sensitivity.

5. During the gradient centrifugation the microorganisms remain freely floating in the solution, which makes it possible to find physiological conditions so that the amount of e.g. metabolites is not changed. Thus, the quantification of the microorganisms can be based also on the analysis of substances with a rapid turnover, e.g. ATP.

6. Gradient centrifugation is an equilibrium technique, i.e. after equilibrium being obtained the time of centrifugation is not critical.

7. Gradient centrifugation has a zone sharpening effect and microorganisms which are reversibly associated with other material will free themselves during the centrifugation and migrate to the microorganism band.

8. The position of the microorganism band in the density gradient could easily be determined by means of special density markers (e.g. Density Marker Beads from Pharmacia Fine Chemicals AB).

9. The methodology is fast and simple. The gradient centrifugation can often be carried out by using a simple bench top centrifuge (400 g, 15—20 minutes). Lysis of non - microbiological cells with e.g. Triton X-100 often takes place momentarily.

The combination of gradient centrifugation and specific lysis of non - microbiological cells as described above thus constitutes a very strong analytical technique applicable in many areas where a rapid analysis of microorganisms has been required for a long time.

The choice of gradient for the gradient centrifugation is easily decided by the experienced laboratory worker (see e.g. Percoll®. Methodology and applications. Density Marker Beads. For calibration of gradients of Percoll Pharmacia Fine Chemicals AB, S-751 04 Uppsala 1, 1980) so that a good separation is obtained and so that the level of the microorganism constituent used for the analysis is not affected during the separation. Continuous as well as discontinuous gradients can be used depending on the application. The gradient material is preferably chosen among such gradients which are known to be mild towards cells, e.g. Percoll (Pharmacia Fine Chemicals AB) possibly with additions to obtain isoosmotic conditions (e.g. 0.15 M NaCl) or other additions which give the microorganisms a suitable physiological surrounding during the separation (e.g. nutritive substances or buffers).

The choice of agents is decided by the types of cells present in the sample and possible interference with the analytical method used. Triton X-100 and saponin are agents which specifically lyse non - microbiological cells but leave the microorganisms unaffected.

The choice of constituents of microorganisms for quantification of microorganisms is decided by the methods of analysis available having adequate sensitivity and specificity. Luminescence analysis of ATP ought to be one of the most attractive analytical principles as both sensitivity and specificity are high. The specificity for microorganisms can furthermore be increased by means of pre-treatment using agents which lyse non - microbiological cells and enzymes or enzyme systems which decompose free ATP. Another alternative could be to let the microorganisms take up some radioactive substance or bring the sample in contact with radioactively marked antibodies, which bind specifically to the microorganism which is to be quantified.

According to this alternative the quantification of the microorganisms is carried out by using radiometric technique (beta- or gamma counters). Other methods of analysis which could be used are e.g. spectrophotometry, fluorimetry, luminometry (ATP or other cell constituents) and visual inspection of the centrifuge tube after centrifuging (the microorganisms become visible as a zone in the centrifuge tube after centrifugation, if the amount is high enough).

The analysis could be carried out either by taking out an aliquote from the centrifuge tube at the density where one knows from earlier experiments that the microorganisms are present or, if this is not known, by taking out aliquotes at different densities or by analysing continously the microorganism constituent along the density gradient (e.g. continuous flow systems). It is also possible to use special density markers (e.g. Density Marker Beads from Pharmacia Fine Chemicals AB). The samples can consist of e.g. blood, urine, wound discharge, milk or cured meats, in which one wants to quantify the presence of microorganisms.

A clinically important application of the invention is the quantification of bacteria in blood when septicaemia (blood-poisoning) is suspected. Septicaemia is a life threatening condition. Therefore, it is desired to be able to confim septicaemia as soon as possible and by determination of antibiotic susceptibility of an isolated bacterium choose a suitable antibiotic treatment.

Today, blood from patients having a suspected septicaemia is inoculated in special blood culture bottles containing a suitable medium for growth of bacteria. These bottles are then incubated at 37°C unhil a growth visible to the eye can be observed (normally at least over night or often for several days). On the thus concentrated bacteria the antibiotic susceptibility determination is then carried out, which requires one further incubation over night when using traditional microbiological technique. Due the slow analysis technique one is often forced to start antibiotic treatment before a response has been received from the laboratory. This means for some patients no doubt wrong or unnecessary and potentially injurious treatment.

According to the embodiment of the invention described in the example below the detection of bacteria in blood can often be carried out the same day as the blood culture bottle arrives at the laboratory. This means that the suspicion of septicaemia could be confirmed one or several days earlier than in conventional methodology. Furthermore, it means that the antibiotic susceptibility determination could be started one or more days earlier. Antibiotic susceptibility determination by means of luminescence technique can be carried out in less than two hours after concentration of an isolated bacterium (Thore, A., Nilsson, L., Höjer H., Ånsehn, S. and Bröte, L. (1977) Acta path. microbiol. scand. Sect. B, 85, 161—166, Höjer, H., Nilsson, L., Ånsehn, S. och Thore, A. (1979) Proceedings from International Symposium on Analytical Applications of Bioluminescence and Chemiluminescence, E. Schram and P. Stanley, Ed., State Printing of Publishing, Inc. Westlake Village, California). This means that in certain cases it could be possible not only to confirm the suspicion of septicaemia but also to choose a suitable antibiotic treatment the same day as the blood culture bottle arrives at the laboratory.

With regard to the fact that in Western Europe and the U.S.A. millions of blood analyses are carried out to confirm septicaemia and the clinical advantages of a rapid analysis especially when combined with a rapid antibiotic susceptibility determination, an analytical system for this application based on the present invention would be of great importance.

Example:

Detection of bacteria in blood.

Production of gradient: 4 ml 31.5% Percoll (Pharmacia) containing 0.15 M NaCl is centrifuged in polycarbonate tubes at 30 000 g during 20 minutes. Thus, a continuous gradient is formed having the density interval 1.14—1.02 g/ml.

Pre-treatment of samples: 1 ml sample from the blood culture bottle is mixed with 1 ml 0.2% Triton X-100 (Eastman-Kodak) containing 0.04% apyrase (grade I, Sigma Chemical Co.) and is incubated at 37°C for 10 minutes.

Gradient centrifugation: The pretreated sample is layered on top of the preprepared gradient and is centrifuged at 400 g for 20 minutes.

After-treatmbnt of samples: 50 µl of the bacterial band (the position of which is known from earlier trials and corresponds to a density of 1.07—1.10 g/ml) is mixed with 50 µl containing 0.04% apyrase and is incubated at 37°C for 10 minutes.

Extraction of bacterial ATP: 50 µl of the aftertreated sample is extracted during 90 s in 500 µl of boiling buffer (0.1 M tris(hydroxymethyl) amino methane+2 mM EDTA, adjusted to pH 7.75 with sulphonic acid).

Luminescence analysis of bacterial ATP: After cooling of the extract 100 µl of luciferase reagent is added (LKB-Wallac ATP Monitoring Reagent, freeze dried reagent reconstituted with 10 ml distilled water) and the light emission is measured in Luminometer 1250 (LKB-Wallac, Åbo, Finland). The ATP concentration is determined compared to an ATP standard of a known concentration which is also analysed according to the instructions from the manufacturer.

**Claims**

1. A method for pre-treatment of samples containing microorganisms and other biological material for use in rapid analysis of microorganisms, characterized in that the sample before the analysis is treated with an agent which lyses specifically non - microbiological cells and that the microorganisms are separated from other biological material by means of equilibrium centrifugation in density gradients.

2. Method according to claim 1, characterized in that non - microbiological cells are lysed before and/or during the equilibrium centrifugation.

3. Method according to claim 1, characterized in that the sample, before, during and/or after gradient centrifugation also is treated with some agent which decomposes specifically the non - microbiological pool of the substance used for quantification of the microorganisms.

4. Method according to claim 1, characterized in that the microorganisms are quantified with some chemical or physical analysis method (e.g. optical or radiometric technique).

5. Method according to claim 1, characterized in that the sample before, during and/or after gradient centrifugation is also treated with some agent which decomposes specifically non - microbiological ATP enzymatically and that the quantification of the microorganisms is based on luminescence analysis of ATP.

## Revendications

1. Procédé de prétraitement d'échantillons contenant des micro-organismes et une autre matière biologique en vue de leur utilisation dans l'analyse rapide de micro-organismes, caractérisé en ce que l'échantillon, avant l'analyse, est traité avec un agent qui effectue la lyse de cellules spécifiquement non microbiologiques et en ce que les micro-organismes sont séparés de l'autre matière biologique au moyen d'une centrifugation en équilibre dans des gradients de densité.

2. Procédé suivant la revendication 1, caractérisé en ce que les cellules non microbiennes sont lysées avant et/ou pendant la centrifugation en équilibre.

3. Procédé suivant la revendication 1, caractérisé en ce que l'échantillon, avant, pendant et/ou après la centrifugation selon des gradients, est également traité avec un agent qui décompose spécifiquement l'ensemble non microbiologique de la substance utilisée pour la détermination quantitative des micro-organismes.

4. Procédé suivant la revendication 1, caractérisé en ce que les micro-organismes sont déterminés quantitativement par une méthode d'analyse chimique ou physique (par exemple par une technique optique ou radiométrique).

5. Procédé suivant la revendication 1, caractérisé en ce que l'échantillon, avant, pendant et/ou après la centrifugation selon des gradients, est également traité avec un agent qui décompose enzymatiquement l'ATP spécifiquement non microbiologique et en ce que la détermination quantitative des micro-organismes est basée sur l'analyse par luminescence de l'ATP.

## Patentansprüche

1. Verfahren zur Vorbehandlung von Mikroorganismen und anderes biologisches Material enthaltende Proben zur Verwendung bei der Schnellanalyse von Mikroorganismen, dadurch gekennzeichnet, daß die Probe vor der Analyse mit einem Agens behandelt wird, das nichtmikrobiologische Zellen speziel zerstört, und daß die Mikroorganismen von dem anderen biologischen Material mit Hilfe von Gleichgewichtszentrifugierung in Dichtegradienten getrennt werden.

2. Verfahren nach Anspruch 1, dadurch gekennzeichnet, daß die nichtmikrobiologischen Zellen vor und/oder während der Gleichgeweichtszentrifugierung zerstört werden.

3. Verfahren nach Anspruch 1, dadurch gekennzeichnet, daß die Probe vor, während und/oder nach der Gradientenzentrifugierung noch mit einem Agens behandelt wird, das das nichtmikrobiologische Becken der Substanz, welches zur quantitiven Bestimmung der Mikroorganismen verwendet wird, speziell zersetzt.

4. Verfahren nach Anspruch 1, dadurch gekennzeichnet, daß die Mikroorganismen nach einer chemischen oder physikalischen Analysemethode (z.B. mit optischer oder radiometrischer Technik) quantitativ bestimmt werden.

5. Verfahren nach Anspruch 1, dadurch gekennzeichnet, daß die Probe vor, während und/oder nach der Gradientenzentrifugierung noch mit einem Agens behandelt wird, welches nichtmikrobiologisches Adenosintriphosphat (ATP) enzymatisch speziell zersetzt, und daß die quantitative Bestimmung der Mikroorganismen auf der Luminiszenzanalyse des ATP basiert.